# EUROPÄISCHE PATENTSCHRIFT

(11) **EP 1 202 983 B1**
(45) Veröffentlichungstag und Bekanntmachung des Hinweises auf die Patenterteilung: **29.01.2003**
(21) Anmeldenummer: 00954620.1
(22) Anmeldetag: 10.08.2000
(51) Int. Cl.: C07D 339/04

(54) **VERFAHREN ZUR HERSTELLUNG VON LÖSEMITTELFREIER ALPHA-LIPONSÄURE**
METHOD FOR THE PRODUCTION OF A SOLVENT-FREE ALPHA- LIPONIC ACID
PROCEDE DE PREPARATION D'ACIDE ALPHA-LIPOIQUE DEPOURVU DE SOLVANT

(30) Priorität: 14.08.1999 DE 19938621
(43) Veröffentlichungstag der Anmeldung: 08.05.2002
(73) Patentinhaber: Degussa AG, 83308 Trostberg (DE)
(72) Erfinder: SCHUHBAUER, Hans, 83308 Trostberg (DE); WINKLER, Stefan, 83119 Obing (DE); GRUBER, Ansgar, 83352 Altenmarkt (DE)
(74) Vertreter: Huber, Bernhard, Dipl.-Chem.
(86) Internationale Anmeldenummer: EP0007802
(87) Internationale Veröffentlichungsnummer: WO01012620

(56) Entgegenhaltungen:
- DE-A- 19 726 519

## Beschreibung

Die vorliegende Erfindung betrifft ein Verfahren zur Herstellung von lösemittelfreier α-Liponsäure.

α-Liponsäure (Thioctsäure, 1,2-Dithiolan-3-pentansäure) ist ein in der Form des R-Enantiomeren in geringen Konzentrationen in pflanzlichen und tierischen Zellen vorkommender Naturstoff. Die ursprünglich als Wuchsfaktor entdeckte α-Liponsäure wirkt physiologisch in hydrophilen und lipophilen Medien als Coenzym der oxidativen Decarboxylierung von α-Ketocarbonsäuren (z.B. Pyruvaten) und als Antioxidans und ist zur Regeneration von Vitamin C, Vitamin E, Glutathion und Coenzym Q10 befähigt. Racemische α-Liponsäure ist zur Behandlung von Lebererkrankungen und Neuropathien (z.B. diabetische Polyneuropathie) zugelassen; ihr Einsatz als effektiver Inhibitor der Replikation von HIV-1-Viren wurde diskutiert (vgl. Klin. Wochenschr. 1991, 69(15), 722-724). Das Racemat von α-Liponsäure weist auch zytoprotektive, antiphlogistische und antinociceptive (analgetische) Eigenschaften auf, wobei sich herausgestellt hat, dass bei den reinen optischen Isomeren der α-Liponsäure (R-α-Liponsäure bzw. S-α-Liponsäure) im Gegensatz zum Racemat das R-Enantiomer ein überwiegend antiphlogistisches und das S-Enantiomer ein überwiegend antinociceptives Wirkungsprofil zeigt (vgl. EP 0 812 590 A2).

Die Synthesen von roher racemischer α-Liponsäure sowie von enantiomerenreiner R- oder S-α-Liponsäure erfolgt nach bekannten oder dazu analogen Verfahren, wie sie beispielsweise in Crévisy et al., Eur. J. Org. Chem. 1998, 1949, Fadnavis et al., Tetrahedron Asym. 1998, 9, 4109, Dhar et al., J. Org. Chem. 1992, 57, 1699, Adger et al., J. Chem. Soc. Chem. Commun. 1995, 1563, Dasaradhi et al., J. Chem. Soc. Chem.

Commun. 1990, 729, Gopalan et al., J. Chem. Soc. Perkin Trans. I 1990, 1897, Yadav et al., J. Sci. Ind. Res. 1990, 49, 400, Tolstikov et. al., Bioorg. Khim. 1990, 16, 1670, Gopalan et al., Tetrahedron Lett. 1989, 5705, beschrieben oder zusammengefasst sind.

Die übliche Reinigungsmethode für rohe α-Liponsäure ist eine Umkristallisation aus Lösemitteln (z.B. aus n-Pentan, Cyclohexan, Methylcyclohexan, Ethylacetat) oder Gemischen von Lösemitteln (z.B. aus Ethylacetat und Hexan), wie sie beispielsweise in Brookes et al., J. Chem. Soc. Perkin Trans. 1 1988, 9, Segre et al., J. Am. Chem. Soc. 1957, 3503, Walton et al., J. Am. Chem. Soc. 1955, 77, 5144, Acker et al., J. Am. Chem. Soc. 1954, 76, 6483, empfohlen wird. Die auskristallisierte α-Liponsäure wird dann abfiltriert oder abzentrifugiert und anschließend mit gängigen Methoden getrocknet. Die so erhaltene kristalline, aber Lösemittelreste enthaltende α-Liponsäure wird schließlich zum fertigen Arzneimittel weiterverarbeitet.

Als alternative zusätzliche Reinigungsmethode für Liponsäure, die zuvor aus einem Gemisch aus Cyclohexan und Ethylacetat umkristallisiert wurde, wird in DE 197 26 519 A1 eine Behandlung des rohen, mit Liponsäure angereicherten Materials mit flüssigem oder überkritischem Kohlendioxid vorgeschlagen. Dennoch weist auch die so erhaltene gereinigte Liponsäure noch Restlösemittelgehalte von 8 bis 1030 ppm Cyclohexan und 83 bis 225 ppm Ethylacetat auf. Darüberhinaus ist das geschilderte Verfahren der Behandlung mit flüssigem oder überkritischem Kohlendioxid in einem Druckbereich von 50 bis 1000 bar aufwendig und nur unter Beachtung entsprechender Sicherheitsmaßnahmen durchführbar.

Wie sich herausgestellt hat, können als Folge des Herstellungsprozesses Restgehalte an Lösemittel in α-Liponsäure nicht völlig ausgeschlossen werden. Nach der 4. Internationalen Harmonisierungskonferenz (ICH 4) sind bestimmte Lösemittel in der Produktion von aktiven Wirkstoffen oder medizinischen Produkten aber entweder gänzlich zu vermeiden (Klasse 1 Lösemittel: mit bekannter oder stark verdächtiger Human-Kanzerogenität, mit umweltgefährdenden Eigenschaften), oder zum Schutz von Patienten vor möglichen adversen Reaktionen zu begrenzen (Klasse 2 Lösemittel: mit nicht-gentoxischer Tier-Kanzerogenität, mit irreversibler Toxizität oder reversibler, aber signifikanter Toxizität). Nach Möglichkeit sollten weniger toxische Lösemittel (Klasse 3) zum Einsatz kommen. Dabei ist diese Klassifizierung keineswegs erschöpfend, und neue wissenschaftliche Erkenntnisse über empfohlene Grenzwerte können ebenso hinzukommen wie sich die Einstufung bestimmter Lösemittel ändern kann. Beispielsweise gibt es Tendenzen, bei α-Liponsäure den Grenzwert des Restlösemittelgehalts für medizinische Zulassungen noch weiter herabzusetzen. Medizinische Wirkstoffe und Produkte sollten in keinem Fall Gehalte an Restlösemitteln enthalten, die sicherheitsrelevante Grenzen überschreiten. Aber auch unkritische Lösemittel, wie z.B. die als Lebensmittelzusatz erlaubte Essigsäure, können auf Geruch oder Geschmack eines Wirkstoffs oder medizinischen Produkts einen unangenehmen Einfluss haben. Da aus einem Restgehalt an Lösemitteln kein therapeutischer Nutzen gezogen werden kann, sollten diese Lösemittel generell so weit es geht vermieden (oder gegebenenfalls entfernt) werden, um den Anforderungen von GMP (Good Manufacturing Practice), Produktspezifikationen und anderen Qualitätsmerkmalen zu genügen. Als Richtlinien bieten sich im Rahmen der oben erwähnten ICH 4 verschiedene Konzepte an, z.B. Richtlinie Q3A, Impurities ih New Active Substances; Q3B, Impurities in New Medicinal Products.

Der vorliegenden Erfindung liegt daher die Aufgabe zu Grunde, ein Verfahren zur Herstellung von lösemittelfreier α-Liponsäure zu schaffen, welches die Nachteile der bekannten Verfahren nicht aufweist, sondern in einfacher Weise die Herstellung von α-Liponsäure ermöglicht, die keine organischen Restlösemittel mehr enthält.

Diese Aufgabe wurde erfindungsgemäß dadurch gelöst, dass man
a) die zu reinigende α-Liponsäure in wässriger, alkalischer Lösung löst oder deren Salz in Wasser löst und einen alkalischen pH-Wert einstellt,
b) evtl. vorhandene feste Verunreinigungen aus der wässrigen Lösung von Stufe a) abtrennt,
c) die wässrige Lösung aus Stufe a) oder b) mit Hilfe einer Säure auf einen pH-Wert von - 1,0 bis 5,0 einstellt und
d) die ausgefällte α-Liponsäure nach bekannten Methoden isoliert.

Es hat sich hierbei überraschenderweise gezeigt, dass man auf diese Weise nicht nur eine lösemittelfreie α-Liponsäure erhält, sondern dass darüber hinaus typische Verunreinigungen aus dem Herstellungsprozess, wie z.B. 1,2,3-Trithian-4-valeriansäure (6,8-Epithioctsäure), abgetrennt werden und so die chemische Reinheit von α-Liponsäure verbessert wird.

Beim Verfahren der Erfindung wird die zu reinigende α-Liponsäure in Stufe a) in wässriger alkalischer Lösung zweckmäßig mit einem pH-Wert von 7,5 bis 16,0, vorzugsweise von 9,0 bis 14,0, gelöst. Die alkalische Lösung kann die üblichen Basen in Form von Hydroxiden, Carbonaten und Hydrogencarbonaten von Alkali- oder Erdalkalimetallen (wie z.B. Natrium, Kalium, Calcium und Magnesium), Ammoniak oder primäre, sekundäre oder tertiäre Amine (wie z.B. Benzylamin, Diisopropylamin, Triethylamin) enthalten.

Die in Stufe a) eingesetzte rohe α-Liponsäure kann hierbei mittels eines beliebigen Verfahrens hergestellt sein. Die α-Liponsäure kann beispielsweise durch Umkristallisation mit einem organischen Lösemittel hergestellt sein oder rohe α-Liponsäure in Abwesenheit von organischen Lösemitteln sein.

Ebenso können beim erfindungsgemäßen Verfahren sowohl racemische α-Liponsäure als auch eine enantiomerenreine R-( + )-α-Liponsäure oder S-(-)-α-Liponsäure bzw. beliebige Mischungen davon eingesetzt werden. Anstelle der freien α-Liponsäure kann auch ein Salz der α-Liponsäure in Wasser gelöst und anschließend alkalisch eingestellt werden, z.B. mit den bereits beschriebenen Basen, zweckmäßig auf einen pH-Wert von 7,5 bis 16,0, vorzugsweise 9,0 bis 14,0.

Gemäß einer bevorzugten Ausführungsform werden hierbei Alkali- (wie z.B. Natrium oder Kalium) oder Erdalkalimetall-Salze (wie z.B. Calcium oder Magnesium) der α-Liponsäure verwendet. Jedoch können auch andere Salze der α-Liponsäure eingesetzt werden, wobei deren Kationen insbesondere aus den Elementen Zink, Eisen, Kupfer, Palladium, Vanadium und Selen bestehen können. Auch α-Liponsäure-Salze von organischen Kationen, wie z.B. offenkettige oder cyclische Ammoniumverbindungen (wie z.B. Ammonium-, Methylammonium-, Benzylmethylammonium- oder Tetramethylammonium-Kationen), komplexen Kationen (mit metallischen Zentralatomen wie z.B. Eisen(III), Chrom(III) oder Cobalt(II) und neutralen, kationischen oder anionischen Liganden (wie z.B. Wasser (H₂O), Ammoniak (NH₃), Carbonyl (CO), Cyano (CN) oder Nitroso(NO)) oder Oxokationen (wie z.B. Oxovanadium(V) (VO₂⁺) oder Oxovanadium(IV) (VO²⁺)) können als Ausgangsverbindungen eingesetzt werden.

Der Gehalt an α-Liponsäure in Stufe a) kann in weiten Grenzen variiert werden. Es hat sich jedoch als vorteilhaft erwiesen, die Konzentration von α-Liponsäure in der wässrigen alkalischen Lösung auf 0,01 bis 15 Gew.-%, bevorzugt auf 0,1 bis 5 Gew.-%, einzustellen.

Gemäß einer bevorzugten Ausführungsform setzt man der wässrigen alkalischen Lösung in Stufe a) vor der Durchführung von Stufe b) Aktivkohle in einer Menge von 0,01 bis 50 Gew.-% bezogen auf die α-Liponsäure-Äquivalente in der Lösung zu. Mit Hilfe dieser Aktivkohlen ist es zusätzlich möglich, störende Verunreinigungen, Nebenprodukte und/oder Restlösemittel abzutrennen. Als besonders vorteilhaft haben sich beispielsweise Aktivkohlen der Marke Norit SX Plus, Norit Pureflow 1, Norit Pureflow C oder Norit SA Plus erwiesen.

In der nachfolgenden Stufe b) werden eventuell vorhandene feste Verunreinigungen aus der wässrigen Lösung von Stufe a) abgetrennt. Geeignet sind die üblichen Methoden wie Filtration, Zentrifugation u.dgl.

Die wässrige Lösung aus Stufe a) oder b) wird in der nachfolgenden Stufe c) mit Hilfe einer Säure auf einen pH-Wert von - 1,0 bis 5,0, bevorzugt von 1,0 bis 4,0 eingestellt. Dies ist erfindungswesentlich. Für die pH-Wert-Einstellung können sowohl anorganische als auch organische Säuren eingesetzt werden. Aus der Gruppe der anorganischen Säuren haben sich die üblichen Mineralsäuren, wie z.B. Halogenwasserstoffsäure (in Form von Salzsäure oder Bromwasserstoffsäure), Salpetersäure, Schwefelsäure und Phosphorsäure besonders gut bewährt. Die pH-Wert-Einstellung kann auch mit organischen Säuren, wie z.B. einer aliphatischen Carbonsäure mit 1 bis 6 Kohlenstoffatomen (z.B. Ameisensäure, Essigsäure), einer aromatischen Carbonsäure (z.B. Benzoesäure), einer halogenierten oder oxygenierten Carbonsäure (z.B. Chloressigsäure, Trifluoressigsäure, Brenztraubensäure) oder einer aliphatischen oder aromatischen Sulfonsäure (z.B. Methansulfonsäure, Toluolsulfonsäure) erfolgen. Durch das Ansäuern fällt die α-Liponsäure aus.

Die Temperatur in den Stufen a) bis c) ist unkritisch, sie wird aber zweckmäßig im Bereich von -50 bis +60 °C gewählt, wobei Raumtemperaturen bevorzugt werden.

In der anschließenden Stufe d) wird die durch die pH-Wert-Einstellung ausgefällte Liponsäure abgetrennt, bevorzugt durch Filtration oder Zentrifugation. Die Temperatur in Stufe d) wird zweckmäßig im Bereich von -50 bis +60 °C gewählt, bevorzugt im Bereich von -10 bis +10 °C.

Die aus Stufe d) anfallende gereinigte α-Liponsäure enthält als einzigen Nebenbestandteil das physiologisch völlig unbedenkliche Solvens Wasser. Die Lagerstabilität von feuchter, halbfeuchter oder trockener α-Liponsäure wird durch den Wassergehalt nicht beeinträchtigt. Falls gewünscht, kann man durch herkömmliche Trocknung, beispielsweise in einem Wirbelschichttrockner, Schaufertrockner, Kegelschnecken-Mischtrockner oder Doppelkonus-Mischtrockner den Wassergehalt der gereinigten und getrockneten α-Liponsäure auf Werte von < 0,5, insbesondere < 0,1 Gew.-% verringern.

In der nach dem erfindungsgemäßen Verfahren hergestellten α-Liponsäure können keine organischen Lösemittel mehr nachgewiesen werden. Nachweis und Bestimmungsgrenzen für eine Reihe von gängigen organischen Lösemitteln, die bei der Herstellung und/oder Reinigung von α-Liponsäure eingesetzt werden können, sind in der nachfolgenden Tabelle 1 beispielhaft zusammengefasst.

**Tabelle 1**

| Nachweisgrenzen und Bestimmungsgrenzen von organischen Lösemitteln | | |
|---|---|---|
| **Lösemittel** | **Nachweisgrenze** | **Bestimmungsgrenze** |
| Acetonitril | 10 ppm | 50 ppm |
| Cyclohexan | 1,2 ppm | 4 ppm |
| Dichlormethan | 7,6 ppm | 25 ppm |
| Ethanol | 5,3 ppm | 25 ppm |
| Ethylacetat | 5,7 ppm | 19 ppm |
| Methanol | 11 ppm | 38 ppm |
| Methylacetat | 1,6 ppm | 5 ppm |
| Methyltertiärbutylether | 10 ppm | 20 ppm |
| Toluol | 5,8 ppm | 19 ppm |

Das erfindungsgemäße Verfahren stellt also eine wesentliche Verbesserung auf dem Gebiet der Reinigung von racemischer oder enantiomerenreiner α-Liponsäure dar, weil den Qualitätsanforderungen an Arzneimittelwirkstoffe und medizinische Produkte durch eine vollständige Entfernung aller Restlösemittelspuren voll Rechnung getragen wird.

Die nachfolgenden Beispiele sollen die Erfindung näher erläutern.

### Beispiele

Für die Messung des Gehalts an Restlösemittel wird die erhaltene α-Liponsäure (ca. 1 g) in 10 ml p-Chlortoluol gelöst und ein Aliquot dieser Lösung direkt in einen Gaschromatographen eingespritzt. Der Nachweis erfolgt per FID (Flammen-lonisations-Detektion) gegen einen externen Standard. Nachweis- und Bestimmungsgrenzen sind in Tabelle 1 zusammengefasst.

### Beispiel 1

30 kg racemischer, kommerzieller α-Liponsäure, die aus einem Gemisch von Ethylacetat und Cyclohexan umkristallisiert wurde und die nach Trocknung in einem Schaufeltrockner einen Restlösemittelgehalt von 1,2 Gew.-% aufwies, wurde bei 20 °C in 1000 I Wasser eingebracht. Mit 50 %-iger wässriger Natronlauge wurde ein pH-Wert von 9,0 eingestellt. Diese verdünnte wässrige Lösung wurde über einen 2 µ-Filter filtriert und bei 0 °C 5 %-iger wässriger Salzsäure auf einen pH-Wert von 1,0 gebracht. Die ausgefallene α-Liponsäure wurde in einer Zentrifuge von der Mutterlauge abgetrennt und mit Wasser solange gewaschen, bis das Waschwasser einen neutralen pH-Wert aufwies. Nach Trocknung in einem Schaufeltrockner (35 °C, 5 mbar, 15 h) wurden die Standard-Parameter einer Analyse des Produkts ermittelt (Tabelle 2).

### Beispiel 2

20 kg racemischer α-Liponsäure, die aus einem Gemisch von Ethylacetat und Cyclohexan umkristallisiert wurde und die nach Trocknung in einem Schaufeltrockner einen Restlösemittelgehalt von 1,2 Gew.-% aufwies, wurde bei 20 °C in 1000 I Wasser eingebracht. Mit 50 %-iger wässriger Natronlauge wurde ein pH-Wert von 9,0 eingestellt, sodann wurden 1,0 kg Aktivkohle vom Typ Norit Pureflow 1 zugegeben. Diese verdünnte wässrige Lösung wurde über einen 2 µ-Filter filtriert und bei Raumtemperatur mit 15 %-iger wässriger Salzsäure auf einen pH-Wert von 2,0 gebracht. Die ausgefallenen α-Liponsäure wurde in einer Zentrifuge von der Mutterlauge abgetrennt und mit Wasser solange gewaschen, bis das Waschwasser einen neutralen pH-Wert aufwies. Nach Trocknung in einem Schaufeltrockner (35 °C, 5 mbar, 15 h) wurden die Standard-Parameter einer Analyse des Produkts ermittelt (Tabelle 2).

### Beispiel 3

30 kg racemischer α-Liponsäure aus einer chemischen Synthese wurde bei 40 °C in 1000 I Wasser eingebracht. Mit wässriger Natronlauge wurde ein pH-Wert von 13,0 eingestellt, sodann wurde 1,0 kg Aktivkohle vom Typ Norit Pureflow 1 zugegeben. Diese verdünnte wässrige Lösung wurde über einen 2 µ-Filter filtriert und bei 40 °C mit 15 %-iger wässriger Salzsäure auf einen pH-Wert von 2,0 gebracht. Die ausgefallene α-Liponsäure wurde in einer Zentrifuge von der Mutterlauge abgetrennt und mit Wasser solange gewaschen, bis das Waschwasser einen neutralen pH-Wert aufwies. Nach Trocknung in einem Schaufeltrockner (40 °C, 5 - 10 mbar, 15 h) wurden die Standard-Analysenparameter des Produkts ermittelt (Tabelle 2).

**Tabelle 2**

| Analysen-Parameter für rohe und gereinigte Liponsäure der Beispiele 1 bis 3 | | | | | |
|---|---|---|---|---|---|
| **α-Liponsäure** | **Schmp.** | **Gehalt**^{**1**} | **Verunreinigungen**^{**1,2**} | **H**_{**2**}**O**^{**3**} | **Restlösemittel**^{**4**} |
| Beispiel 1: rohe Liponsäure | 59,6 - 61,3 °C | 99,0 % | < 1,0 % | 0,5 % | CH₂Cl₂: 880 ppm |
| | | | | | Ethanol: 540 ppm |
| | | | | | MTBE: 650 ppm |
| | | | | | unbekannt: 590 ppm⁵ |
| Beispiel 1: reine Liponsäure | 60,1 - 61,1 °C | 99,1 % | < 1,0 % | 0,1 % | Acetonitril: n.n. |
| | | | | | Cyclohexan: n.n. |
| | | | | | CH₂Cl₂: n.n. |
| | | | | | Ethanol: n.n. |
| | | | | | Ethylacetat: n.n. |
| | | | | | Methanol: n.n. |
| | | | | | Methylacetat: n.n. |
| | | | | | MTBE: n.n. |
| | | | | | Toluol: n.n. |
| Beispiel 2: rohe Liponsäure | 60,3 °C | 96,9 % | < 1,0 % | 0,5 % | Cyclohexan: 2950 ppm |
| | | | | | Ethylacetat: 420 ppm |
| Beispiel 2: reine Liponsäure | 60,5 - 61,7 °C | 99,7 % | < 1,0 % | 0,1 % | Acetonitril: n.n. |
| | | | | | Cyclohexan: n.n. |
| | | | | | CH₂Cl₂: n.n. |
| | | | | | Ethanol: n.n. |
| | | | | | Ethylacetat: n.n. |
| | | | | | Methanol: n.n. |
| | | | | | Methylacetat: n.n. |
| | | | | | MTBE: n.n. |
| | | | | | Toluol: n.n. |
| Beispiel 3: rohe Liponsäure | 60,0 - 61,0 °C | 96,5 % | < 1,0 % | 0,3 % | Cyclohexan: 550 ppm |
| | | | | | Methanol: 120 ppm |
| | | | | | Toluol: 335 ppm |
| Beispiel 3: reine Liponsäure | 60,4 - 61,5 °C | 99,9 % | < 1,0 % | 0,05 % | Acetonitril: n.n. |
| | | | | | Cyclohexan: n.n. |
| | | | | | CH₂Cl₂: n.n. |
| | | | | | Ethanol: n.n. |
| | | | | | Ethylacetat: n.n. |
| | | | | | Methanol: n.n. |
| | | | | | Methylacetat: n.n. |
| | | | | | MTBE: n.n. |
| | | | | | Toluol: n.n. |

| | | | | | |
|---|---|---|---|---|---|
| ¹ per HPLC | | | | | |
| ² z.B. 6,8-Epiliponsäure | | | | | |
| ³ per Karl-Fischer-Titration | | | | | |
| ⁴ n.n. = nicht nachweisbar | | | | | |
| ⁵ nicht zugeordneter Lösemittel-Peak, als Cyclohexan ausgewertet | | | | | |

## Patentansprüche

1. Verfahren zur Herstellung von lösemittelfreier α-Liponsäure,
**dadurch gekennzeichnet,**
**dass** man
a) die zu reinigende α-Liponsäure in wässriger, alkalischer Lösung löst
oder deren Salze in Wasser löst und einen alkalischen pH-Wert einstellt,
b) gegebenenfalls vorhandene feste Verunreinigungen aus der wässrigen Lösung von Stufe a) abtrennt,
c) die wässrige Lösung aus Stufe a) oder b) mit Hilfe einer Säure auf einen pH-Wert von -1,0 bis 5,0 einstellt und
d) die ausgefällte α-Liponsäure abtrennt.

2. Verfahren nach Anspruch 1,
**dadurch gekennzeichnet,**
**dass** man eine racemische α-Liponsäure oder eine enantiomerenreine R-(+)-α-Liponsäure oder S-(-)-α-Liponsäure bzw. Mischungen davon einsetzt.

3. Verfahren nach Anspruch 1 oder 2,
**dadurch gekennzeichnet,**
**dass** die wässrige alkalische Lösung in Stufe a) einen Gehalt an α-Liponsäure von 0,01 bis 15,0 Gew.-% aufweist.

4. Verfahren nach einem der Ansprüche 1 bis 3,
**dadurch gekennzeichnet,**
**dass** die alkalische Lösung als Base Hydroxide, Carbonate, Hydrogencarbonate von Alkali- oder Erdalkalimetallen, Ammoniak oder primäre, sekundäre oder tertiäre Amine enthält.

5. Verfahren nach einem der Ansprüche 1 bis 4,
**dadurch gekennzeichnet,**
**dass** man ein Alkali- oder Erdalkalimetall-Salz der α-Liponsäure verwendet.

6. Verfahren nach einem der Ansprüche 1 bis 4,
**dadurch gekennzeichnet,**
**dass** die Salze der α-Liponsäure Kationen, ausgewählt aus den Elementen Zink, Eisen, Kupfer, Palladium, Vanadium und Selen enthalten.

7. Verfahren nach einem der Ansprüche 1 bis 4,
**dadurch gekennzeichnet,**
**dass** die α-Liponsäure-Salze organische Kationen, insbesondere offenkettige oder cyclische Ammoniiumverbindungen, komplexe Kationen oder Oxokationen enthalten.

8. Verfahren nach einem der Ansprüche 1 bis 7,
**dadurch gekennzeichnet,**
**dass** der pH-Wert in Stufe a) 7,5 bis 16,0 beträgt.

9. Verfahren nach Anspruch 8,
**dadurch gekennzeichnet,**
**dass** der pH-Wert in Stufe a) auf 9,0 bis 14,0 eingestellt wird.

10. Verfahren nach einem der Ansprüche 1 bis 9,
**dadurch gekennzeichnet,**
**dass** man der wässrigen alkalischen Lösung in Stufe a) vor der Durchführung von Stufe b) Aktivkohle in einer Menge von 0,01 bis 50 Gew.-%, bezogen auf die α-Liponsäure-Äquivalente in der Lösung, zusetzt.

11. Verfahren nach einem der vorhergehenden Ansprüche,
**dadurch gekennzeichnet,**
**dass** man in Stufe b) die Abtrennung evtl. vorhandener fester Verunreinigungen durch Filtration vornimmt.

12. Verfahren nach einem der vorhergehenden Ansprüche,
**dadurch gekennzeichnet,**
**dass** man die pH-Wert-Einstellung in Stufe c) mit einer Mineralsäure, ausgewählt aus der Gruppe Halogenwasserstoffsäure, Salpetersäure, Schwefelsäure und Phosphorsäure vornimmt.

13. Verfahren nach Anspruch 12,
**dadurch gekennzeichnet,**
**dass** man als Halogenwasserstoffsäure Salzsäure oder Bromwasserstoffsäure verwendet.

14. Verfahren nach einem der Ansprüche 1 bis 11,
**dadurch gekennzeichnet,**
**dass** man die pH-Einstellung in Stufe c) mit einer aliphatischen Carbonsäure mit 1 bis 6 Kohlenstoffatomen, einer aromatischen Carbonsäure, einer halogenierten oder oxygenierten Carbonsäure oder einer aliphatischen oder aromatischen Sulfonsäure vornimmt.

15. Verfahren nach einem der vorhergehenden Ansprüche,
**dadurch gekennzeichnet,**
**dass** die ausgefällte α-Liponsäure in Stufe d) durch Filtration oder Zentrifugation abgetrennt wird.

16. Verfahren nach einem der vorhergehenden Ansprüche,
**dadurch gekennzeichnet,**
**dass** man die Stufen a) bis c) bei Temperaturen von -50 bis + 60 °C, insbesondere bei Raumtemperatur durchführt.

17. Verfahren nach einem der vorhergehenden Ansprüche,
**dadurch gekennzeichnet,**
**dass** man die Stufe d) bei Temperaturen von -50 bis +40 °C und insbesondere im Bereich von -10 bis +10 °C durchführt.

18. Verfahren nach einem der vorhergehenden Ansprüche,
**dadurch gekennzeichnet,**
**dass** die α-Liponsäure nach Stufe d) bis auf einen Wassergehalt < 0,5 Gew.-%, insbesondere < 0,1 Gew.-%, getrocknet wird.

## Claims

1. Process for the production of solvent-free α-lipoic acid,
**characterized in that**
a) the α-lipoic acid to be purified is dissolved in an aqueous alkaline solution
or its salts are dissolved in water and an alkaline pH is adjusted,
b) solid impurities that may be present are removed from the aqueous solution from step a),
c) the aqueous solution from step a) or b) is adjusted to a pH of -1.0 to 5.0 with the aid of an acid and
d) the precipitated α-lipoic acid is separated.

2. Process as claimed in claim 1,
**characterized in that**
a racemic α-lipoic acid or an enantiopure R-(+)-α-lipoic acid or S-(-)-α-lipoic acid or mixtures thereof is used.

3. Process as claimed in claim 1 or 2,
**characterized in that**
the aqueous alkaline solution in step a) has an α-lipoic acid content of 0.01 to 15.0 % by weight.

4. Process as claimed in one of the claims 1 to 3,
**characterized in that**
the alkaline solution contains hydroxides, carbonates, bicarbonates of alkali metals or alkaline earth metals, ammonia or primary, secondary or tertiary amines as the base.

5. Process as claimed in one of the claims 1 to 4,
**characterized in that**
an alkaline metal or alkaline earth metal salt of α-lipoic acid is used.

6. Process as claimed in one of the claims 1 to 4,
**characterized in that**
the salts of α-lipoic acid contain cations selected from the elements zinc, iron, copper, palladium, vanadium and selenium.

7. Process as claimed in one of the claims 1 to 4,
**characterized in that**
the α-lipoic acid salts contain organic cations, in particular open-chain or cyclic ammonium compounds, complex cations or oxo cations.

8. Process as claimed in one of the claims 1 to 7,
**characterized in that**
the pH in step a) is 7.5 to 16.0.

9. Process as claimed in claim 8,
**characterized in that**
the pH in step a) is adjusted to 9.0 to 14.0.

10. Process as claimed in one of the claims 1 to 9,
**characterized in that**
activated carbon is added in an amount of 0.01 to 50 % by weight, based on the α-lipoic acid equivalent in the solution, to the aqueous alkaline solution in step a) before step b) is carried out.

11. Process as claimed in one of the previous claims,
**characterized in that**
solid impurities that may be present in step b) are removed by filtration.

12. Process as claimed in one of the previous claims,
**characterized in that**
the pH is adjusted in step c) with a mineral acid selected from the group of hydrohalic acid, nitric acid, sulphuric acid and phosphoric acid.

13. Process as claimed in claim 12,
**characterized in that**
hydrochloric acid or hydrobromic acid is used as the hydrohalic acid.

14. Process as claimed in one of the claims 1 to 11,
**characterized in that**
the pH is adjusted in step c) with an aliphatic carboxylic acid having 1 to 6 carbon atoms, an aromatic carboxylic acid, a halogenated or oxygenated carboxylic acid or an aliphatic or aromatic sulphonic acid.

15. Process as claimed in one of the previous claims,
**characterized in that**
the precipitated α-lipoic acid is separated in step d) by filtration or centrifugation.

16. Process as claimed in one of the previous claims,
**characterized in that**
steps a) to c) are carried out at temperatures of -50 to +60°C, in particular at room temperature.

17. Process as claimed in one of the previous claims,
**characterized in that**
step d) is carried out at temperatures of -50 to +40°C and in particular in the range from -10 to +10°C.

18. Process as claimed in one of the previous claims,
**characterized in that**
the α-lipoic acid is dried after step d) until the water content is < 0.5 % by weight, in particular < 0.1 % by weight.

## Revendications

1. Procédé de préparation d'acide α-lipoïque dépourvu de solvant, **caractérisé en ce que** l'on
a) dissous l'acide α-lipoïque à purifier dans la solution aqueuse alcaline
ou dissous son sel et ajuste à un pH alcalin,
b) sépare les impuretés solides éventuellement présentes de la solution de l'étape a),
c) ajuste la solution aqueuse de l'étape a) ou b) à l'aide d'un acide à un pH situé entre -1,0 et 5,0 et
d) sépare l'acide α-lipoïque précipité.

2. Procédé selon la revendication 1, **caractérisé en ce que** l'on met en oeuvre un acide α-lipoïque racémique ou un acide α-lipoïque énantiomère pur R-(+) ou S-(-) ou des mélanges de ceux-ci.

3. Procédé selon la revendication 1 ou 2, **caractérisé en ce que** la solution alcaline aqueuse de l'étape a) présente une teneur en acide α-lipoïque allant de 0,01 à 15,0 % en poids.

4. Procédé selon l'une des revendications 1 à 3, **caractérisé en ce que** la solution alcaline contient comme base de l'hydroxyde, du carbonate, de l'hydrogénocarbonate de métaux alcalins et alcalino-terreux, de l'ammoniac ou des amines primaires, secondaires ou tertiaires.

5. Procédé selon l'une des revendications 1 à 4, **caractérisé en ce que** l'on utilise un sel de métal alcalin ou alcalino-terreux de l'acide α-lipoïque.

6. Procédé selon l'une des revendications 1 à 4, **caractérisé en ce que** les sels d'acide α-lipoïque contiennent des cations choisis parmi les éléments zinc, fer, cuivre, palladium, vanadium et sélénium.

7. Procédé selon l'une des revendications 1 à 4, **caractérisé en ce que** les sels d'acide α-lipoïque contiennent des cations organiques, en particulier des composés d'ammonium à chaîne ouverte ou cyclique, des cations complexes ou des oxocations.

8. Procédé selon l'une des revendications 1 à 7, **caractérisé en ce que** le pH atteint 7,5 à 16,0 à l'étape a).

9. Procédé selon la revendication 8, **caractérisé en ce que** le pH est ajusté entre 0,9 et 14,0 à l'étape a).

10. Procédé selon l'une des revendications 1 à 9, **caractérisé en ce que** l'on ajoute à la solution aqueuse alcaline des charbons actifs en une quantité allant de 0,01 à 50 % en poids, sur les équivalents d'acide α-lipoïque en solution, à l'étape a) avant la réalisation de l'étape b).

11. Procédé selon l'une des revendications précédentes, **caractérisé en ce que** l'on réalise la séparation des impuretés solides éventuellement présentes par filtration à l'étape b).

12. Procédé selon l'une quelconque des revendications précédentes, **caractérisé en ce que** l'on ajuste le pH dans l'étape c) avec un acide minéral, choisi parmi le groupe des haloacides, des acides nitriques, des acides sulfuriques et acides phosphoriques.

13. Procédé selon la revendication 12, **caractérisé en ce que** l'on utilise l'acide chlorhydrique ou l'acide bromhydrique comme haloacide.

14. Procédé selon l'une quelconque des revendications 1 à 11, **caractérisé en ce que** l'on ajuste le pH avec un acide carboxylique aliphatique ayant de 1 à 6 atomes de carbone, un acide carboxylique aromatique, un acide carboxylique halogéné ou oxygéné ou un acide sulfonique aliphatique ou aromatique à l'étape c).

15. Procédé selon l'une quelconque des revendications précédentes, **caractérisé en ce que** l'on sépare, à l'étape d), l'acide α-lipoïque précipité par filtration ou centrifugation.

16. Procédé selon l'une quelconque des revendications précédentes, **caractérisé en ce que** l'on réalise les étapes a) à c) à des températures allant de -50 à +60°C, en particulier à température ambiante.

17. Procédé selon l'une quelconque des revendications précédentes, **caractérisé en ce que** l'on réalise l'étape d) à des températures allant de -50 à +40°C et en particulier dans la zone allant de -10 à +10°C.

18. Procédé selon l'une quelconque des revendications précédentes, **caractérisé en ce que** l'acide α-lipoïque est séché après l'étape d) jusqu'à ce qu'il ait une teneur en eau < 0,5 % en poids, notamment < 0,1 % en poids.
